# EUROPEAN PATENT APPLICATION

(11) **EP 4 691 549 A1**
(43) Date of publication of application: **11.02.2026**
(21) Application number: 24793022.5
(22) Date of filing: 18.04.2024
(51) Int. Cl.: A61N 1/40, A61N 1/08, A61N 1/36, A61N 7/02, A61N 7/00

(54) **SKIN CARE DEVICE AND CARE METHOD THEREOF**

(30) Priority: 21.04.2023 KR 20230052397; 17.04.2024 KR 20240051464
(71) Applicant: Jeisys Medical Inc., Seoul 08501 (KR)
(72) Inventor: PARK, Ji Hoon, Seoul 08501 (KR); GO, In Jae, Seoul 08501 (KR)
(74) Representative: dompatent
(86) International application number: PCT/KR2024/005195
(87) International publication number: WO 2024/219837

(57) **Abstract**

The present disclosure may include a main body; a hand piece; and a processor configured to, based on an amount of pressing of a skin care tip and an amount of contact with a skin detected through the hand piece, determine a contact state of the skin and a pressing state of the skin care tip.

## Description

### [TECHNICAL FIELD]

The present disclosure relates to a skin care device and a method therefor.

### [BACKGROUND ART]

The conventional skin care devices generate heat by energy output to the skin tissue of the energy irradiation target, which causes collagen fiber degeneration and contraction, thereby producing collagen regeneration and wrinkle improvement effects.

In such of the conventional skin care devices, when only a portion of the entire electrode is in contact with the skin and energy is irradiated, a large amount of energy is transmitted to a narrow area, which can cause burn accidents.

In addition, the conventional skin care devices cannot accurately determine the contact and pressing state of the skin, so there is a limit to transmit optimal energy to the skin, and there is a limit to preventing the occurrence of burn accidents in advance.

Therefore, recently, research has been continuously conducted on improved devices that can accurately determine the contact and pressing state of the skin, transmit optimal energy to the skin, and prevent the occurrence of burn accidents in advance.

### [DETAILED DESCRIPTION OF THE INVENTION]

### [TECHINICAL PROBLEM]

The present disclosure is to provide a device capable of accurately determining the contact and pressing state of the skin, thereby transmitting optimal energy to the skin and preventing the occurrence of burn accidents in advance.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, a skin care device may include a main body; a hand piece; and a processor configured to, based on an amount of pressing of a skin care tip and an amount of contact with a skin detected through the hand piece, determine a contact state of the skin and a pressing state of the skin care tip.

Furthermore, the hand piece may include: a housing; the skin care tip coupled to the housing; a first sensing module provided on the skin care tip and configured to detect the amount of contact with the skin; and a second sensing module provided on the housing or the skin care tip and configured to detect the amount of pressing of the skin care tip.

Furthermore, the processor may be provided on at least one of the hand piece or the main body, and the processor may be configured to determine the contact state of the skin and the pressing state of the skin care tip based on the detected amount of contact and the amount of pressing.

Furthermore, the first sensing module may be a temperature sensor.

Furthermore, the second sensing module may be at least one of a pressure sensor or a photo interrupter sensor.

Furthermore, the device may further include a notification module configured to notify the determined contact state of the skin and the determined pressing state of the skin care tip.

Furthermore, the notification module may be provided in at least one of the hand piece or the main body.

Furthermore, the processor may be configured to extract at least one of a preset RF energy intensity or a preset ultrasonic energy intensity based on and linked with the detected contact amount and the detected pressing amount, and the hand piece may further include an energy irradiation module configured to irradiate at least one of the RF energy and the ultrasonic energy based on at least one of the extracted RF energy intensity and the extracted ultrasonic energy intensity.

Furthermore, in another aspect of the present disclosure, a skin care method performed by a skin care device may include detecting, by a hand piece of the skin care device, an amount of pressing of a skin care tip and an amount of contact with a skin; receiving, by the hand piece, the detected amount of pressing of the skin care tip and the detected amount of contact with the skin; and determining, by a processor of the skin care device, the contact state of the skin and the pressing state of the skin care tip based on the detected amount of contact and the detected amount of pressing.

Furthermore, detecting the amount of pressing of the skin care tip and the amount of contact with the skin may include: detecting, by a first sensing module provided on the skin care tip of the hand piece, the amount of contact with the skin; and detecting, by a second sensing module provided on the housing of the hand piece or the skin care tip, the amount of pressing of the skin care tip.

Furthermore, determining the contact state of the skin and the pressing state of the skin care tip may further include: determining, by the processor provided on at least one of the hand piece or a main body of the skin care device, the contact state of the skin and the pressing state of the skin care tip based on the detected amount of contact and the detected amount of pressing.

Furthermore, detecting the amount of pressing of the skin care tip and the amount of contact with the skin may include: detecting, by a temperature sensor of the hand piece, the amount of contact with the skin.

Furthermore, detecting the amount of pressing of the skin care tip and the amount of contact with the skin may include: detecting, by at least one of a pressure sensor or a photo interrupter sensor of the hand piece, the amount of pressing of the skin care tip.

Furthermore, the method may further include notifying, by a notification module of the skin care device, the determined contact state of the skin and the determined pressing state of the skin care tip.

Furthermore, the method may further include extracting, by the processor, at least one of a preset RF energy intensity or a preset ultrasonic energy intensity linked with the detected amount of contact and the detected amount of pressing; and controlling, by the processor, an energy irradiation module of the hand piece to irradiate at least one of the RF energy or the ultrasonic energy based on at least one of the extracted RF energy intensity or the extracted ultrasonic energy intensity.

Furthermore, a computer program stored in a computer-readable recording medium for executing the present disclosure may be further provided.

Furthermore, a computer-readable recording medium recording a computer program for executing a method for executing the present disclosure may be further provided.

### [ADVANTAGEOUS EFFECTS OF THE INVENTION]

The present disclosure provides an effect that the contact and pressing state of the skin can be accurately determined, and optimal energy can be transmitted to the skin, thereby preventing the occurrence of a burn accident in advance.

The effects of the present disclosure are not limited to the effects mentioned above, and other effects not mentioned will be clearly understood by those skilled in the art from the description.

### [DESCRIPTION OF THE DRAWINGS]

FIG. 1 is a diagram illustrating an example of a skin care device according to the present disclosure.
FIGS. 2 and 3 are diagrams illustrating an example of a configuration of a hand piece of a skin care device according to the present disclosure.
FIG. 4 is a diagram illustrating an example of a first sensing module provided in the skin care tip illustrated in FIG. 1.
FIG. 5 is a diagram illustrating an example of a second sensing module provided in the skin care tip illustrated in FIG. 2.
FIG. 6 is a block diagram illustrating a hand piece of a skin care device according to the present disclosure.
FIG. 7 is a block diagram illustrating a main body of a skin care device according to the present disclosure.
FIGS. 8 to 10 are flowcharts illustrating an example of an operation method of a skin care device according to the present disclosure.

### [BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "module, module, member, and block" may be embodied as hardware or software, and a plurality of "modules, modules, members, and blocks" may be implemented as one element, or a module, a module, a member, or a block may include a plurality of elements.

Throughout this specification, when a part is referred to as being "connected" to another part, this includes "direct connection" and "indirect connection", and the indirect connection may include connection via a wireless communication network. Furthermore, when a certain part "includes" a certain element, other elements are not excluded unless explicitly described otherwise, and other elements may in fact be included.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for convenience of description, and the operations may be implemented differently from the order described unless there is a specific order explicitly described in the context.

Hereinafter, operation principles and embodiments of the present disclosure will be described with reference to the accompanying drawings.

The control module according to the present disclosure in this specification includes various devices that may perform computational processing and provide results to a user. For example, the control module according to the present disclosure may include a computer, a server device, and a portable terminal, or may be in the form of one of them.

Here, the computer may include, for example, a notebook, a desktop, a laptop, a tablet PC, a slate PC, and the like mounted with a web browser.

The server device is a server that communicates with an external device to process information, and may include an application server, a computing server, a database server, a file server, a mail server, a proxy server, and a web server.

A portable terminal is a wireless communication device that ensures portability and mobility, and may include all kinds of handheld-based wireless communication devices such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), WiBro (Wireless Broadband Internet) terminal, a smart phone, and the like, and a wearable device such as at least one of a watch, a ring, bracelets, anklets, a necklace, glasses, contact lenses, or a head-mounted device (HMD).

A skin care device and a method therefor according to the present disclosure may include detecting an amount of pressing of a skin care tip and an amount of contact with a skin, receiving the detected amount of pressing of the skin care tip and the detected amount of contact with the skin; and determining the contact state of the skin and the pressing state of the skin care tip based on the detected amount of contact and the detected amount of pressing.

The skin care device and the method therefor according to the present disclosure may accurately determine the contact and pressing state of a skin, thereby preventing the occurrence of a burn accident while transmitting optimal energy to the skin.

Hereinafter, the skin care device and the method therefor will be described in detail.

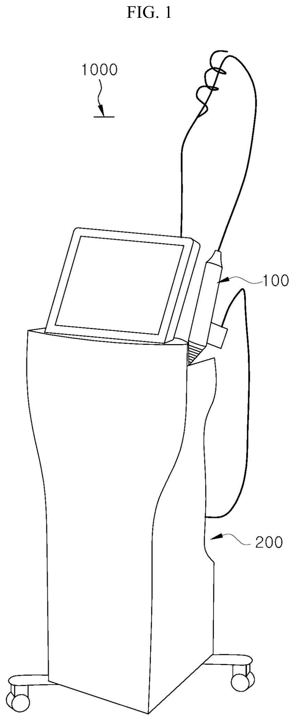

FIG. 1 is a diagram illustrating an example of a skin care device according to the present disclosure.

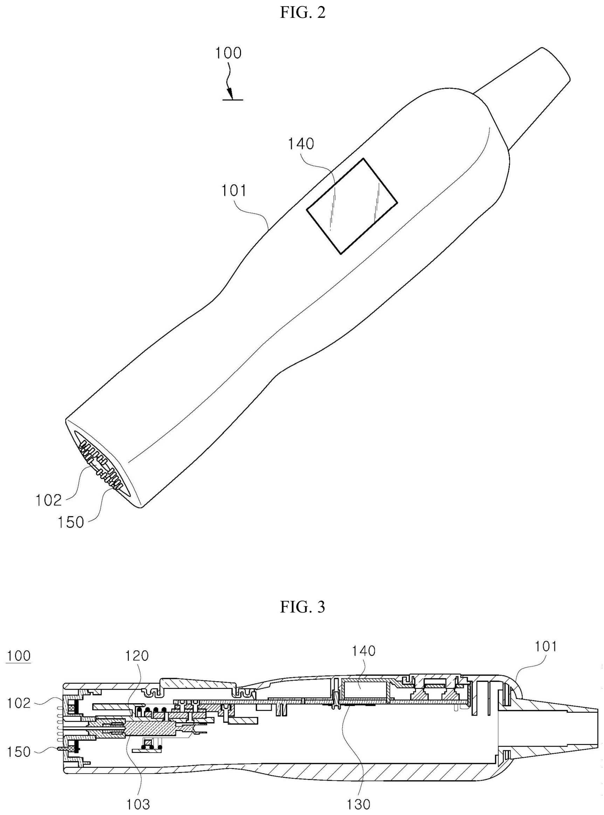

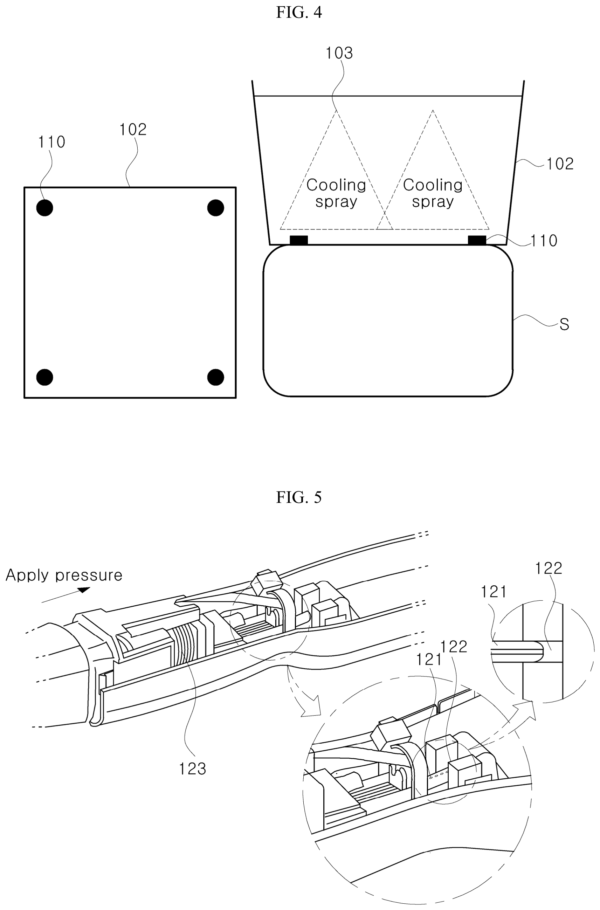

FIGS. 2 and 3 are diagrams illustrating an example of a configuration of a hand piece of a skin care device according to the present disclosure. FIG. 4 is a diagram illustrating an example of a first sensing module provided in the skin care tip illustrated in FIG. 1.

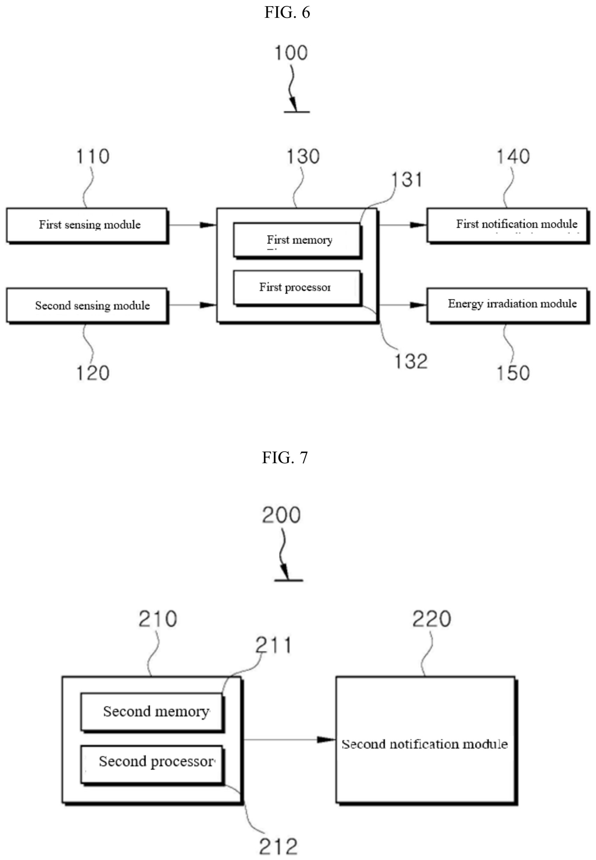

FIG. 5 is a diagram illustrating an example of a second sensing module provided in the skin care tip illustrated in FIG. 2. FIG. 6 is a block diagram illustrating a hand piece of a skin care device according to the present disclosure.

FIG. 7 is a block diagram illustrating a main body of a skin care device according to the present disclosure.

Referring to FIGS. 1 to 7, a hand piece 100 may include a housing 101, a skin care tip 102, a first sensing module 110, a second sensing module 120, and a first control module 130.

The skin care tip 102 may be provided to be coupled with the housing 101, and the first sensing module 110 is provided in the skin care tip 102 and may detect an amount of contact with a skin S. In this case, the first sensing module 110 may be at least one of a temperature sensor, a capacitance sensor, or a contact sensor for detecting the amount of contact with the skin S, and may preferably be a temperature sensor. However, the first sensing module 110 may be any member for detecting a state of contact or the amount of contact with the skin.

Here, as shown in FIG. 3, the first sensing module 110 may be provided at least one at a corner or edge of the skin care tip 102. In this case, a spray module 103, when ready for energy irradiation, may spray a cooling gas at a temperature higher than a specific temperature to lower a temperature of the skin care tip 102 in advance before the energy irradiation. That is, the spray module 103 may instantly lower the internal temperature of the skin care tip 102 and the temperature of the first sensing module 110 by spraying the cooling gas. At this time, the spray module 103 may also arbitrarily spray the cooling gas depending on the temperature conditions.

The second sensing module 120 is provided in the housing 101 or the skin care tip 102 and may detect an amount of pressing of the skin care tip 102. Here, the second sensing module 120 may be at least one of a pressure sensor, a photo interrupter sensor, or a switch for detecting the amount of pressing of the skin care tip 102, and may preferably be a pressure sensor. However, without being limited thereto, the second sensing module 120 may be any member that may detect whether the skin is pressed or the amount of pressing. At this time, the photo interrupter sensor may be a sensor that combines a light emitting diode and a phototransistor.

In addition, as illustrated in FIG. 5, the second sensing module 120 may include a moving bar 121, an infrared sensor 122, and an elastic member 123. The second sensing module 120 may detect that the skin care tip 102 is pressed when the moving bar 121 moves back when the skin care tip 102 contacts with the skin S and a pressure is applied, and when the moving bar 121 covers the center of the infrared sensor 122. In addition, when the pressure of the skin care tip 102 pressing the skin S disappears, the second sensing module 120 moves the skin care tip 102 forward by the elastic member 123, and the moving bar 121 covering the infrared sensor 122 moves forward, so that the infrared sensor 122 may detect that the skin care tip 102 is moved forward.

Without being limited thereto, the second sensing module 120 may be a light sensor, a force sensor, or any member for detecting the amount of pressing of the skin care tip 102. Since the second sensing module 120 is provided on one side of the inside of the housing 101, the manufacturing cost of the skin care tip 102 may be reduced.

The first control module 130 is provided in the housing 101 and may determine the contact state of the skin S and the pressing state of the skin care tip 102 based on the detected amount of contact and the detected amount of pressing. Here, the first control module 130 may analyze a change in temperature acquired by the first sensing module 110 after the cooling gas is sprayed by the spray module 103 and may determine the contact state of the skin S based on the analysis result. At this time, the first control module 130 may determine that the first sensing module 110 is not in contact with the skin S in the case that the change in temperature is small, and may determine that the first sensing module 110 is in contact with the skin S in the case that the change in temperature is great.

Here, the first control module 130 may be manufactured as a printed circuit board. In this case, the first control module 130 may be implemented as a first memory 131 that stores data on an algorithm for controlling the operation of components within the device or a program that reproduces the algorithm, and at least one first processor 132 that performs the operation described above using the data stored in the first memory 131. Here, the first memory 131 and the first processor 132 may each be implemented as separate chips. In addition, the first memory 131 and the first processor 132 may be implemented as a single chip.

The first memory 131 may store data supporting various functions of the device, a program for the operation of the control module, may store input/output data, and may store a plurality of application programs or applications run on the device, data for the operation of the device, and commands. At least some of these application programs may be downloaded from an external server via wireless communication.

The first memory 131 may include at least one type of storage medium among a flash memory type, a hard disk type, an SSD type (Solid State Disk type), an SDD type (Silicon Disk Drive type), a multimedia card micro type, a card type memory (e.g., an SD or XD memory, etc.), random access memory (RAM), static random access memory (SRAM), read-only memory (ROM), electrically erasable programmable read-only memory (EEPROM), programmable read-only memory (PROM), a magnetic memory, a magnetic disk, and an optical disk. In addition, the first memory 131 may be a database that is separate from the device but connected by wire or wirelessly.

The first memory 131 may store data related to the contact state and the pressing state of the skin S. The first processor 132 may control the operation related to the contact state and the pressing state of the skin S. At this time, the first processor 132 may determine the contact state of the skin S and the pressing state of the skin care tip 102 based on the detected amount of contact and the detected amount of pressing. Here, the first processor 132 may determine whether the energy irradiation module 150 including the film-shaped F-PCB electrode provided on the skin care tip 102 is properly in contact with the skin S and properly pressing the skin care tip 102 based on the four first sensing modules 110. At this time, the first processor 132 may determine whether pressure is applied to minimize an influence of a gel applied to the skin S.

The hand piece 100 according to the present disclosure may include a first notification module 140 and an energy irradiation module 150.

The first notification module 140 may notify the determined contact state of the skin S and the determined pressing state of the skin care tip 102. In this case, the notification module 140 may visually notify the contact state of the skin S and the pressing state of the skin care tip 102 and may notify audibly. For example, in the case that the contact state of the skin S is a proper contact state and the pressing state of the skin care tip 102 is a proper pressing state, the first notification module 140 may notify the proper contact and pressing state by at least one of a display device, a light emitting device, or a speaker.

The energy irradiation module 150 may irradiate at least one of an RF energy or an ultrasonic energy with at least one of the corresponding RF energy intensity and the corresponding ultrasonic energy intensity corresponding to a level of the detected amount of contact and a level of the amount of pressing. In this case, the energy irradiation module 150 may be provided on the skin care tip 102 and may include a film-shaped F-PCB electrode for irradiating RF energy or another type of electrode for irradiating ultrasonic energy.

As shown in FIG. 7, the main body 200 may include a second control module 210 and a second notification module 220.

The second control module 210 may include a second memory 211 and a second processor 212.

The second memory 211 may store data related to the contact and pressing state of the skin S. The second processor 212 may control an operation related to the contact and pressing state of the skin S.

At this time, the second processor 212 may detect the amount of contact between the skin S and the amount of pressure of the skin care tip 102 detected through the hand piece 100. The contact state and the pressing state of the skin care tip 102 may be determined.

Here, the second processor 212 may determine whether the energy irradiation module 150 including the film-shaped F-PCB electrode provided on the skin care tip 102 is properly in contact with the skin S and properly pressing the skin care tip 102. At this time, the second processor 212 may determine whether pressure is applied to minimize the influence of the gel applied to the skin S.

The second notification module 220 may notify the determined contact state of the skin S and the pressing state of the skin care tip 102. In this case, the second notification module 220 may visually notify the contact state of the skin S and the pressing state of the skin care tip 102 and may audibly notify. For example, the second notification module 220 may notify the state of proper contact and proper pressing of the skin care tip 102 by at least one of a display device, a light emitting device, and a speaker when the contact state of the skin S is a proper contact state and the pressing state of the skin care tip 102 is a proper pressing state.

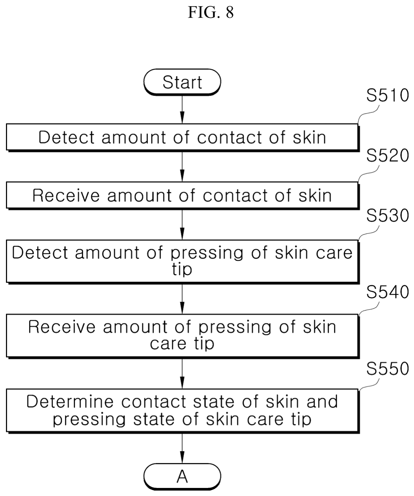

FIGS. 8 to 10 are flowcharts illustrating an example of an operation method of a skin care device according to the present disclosure.

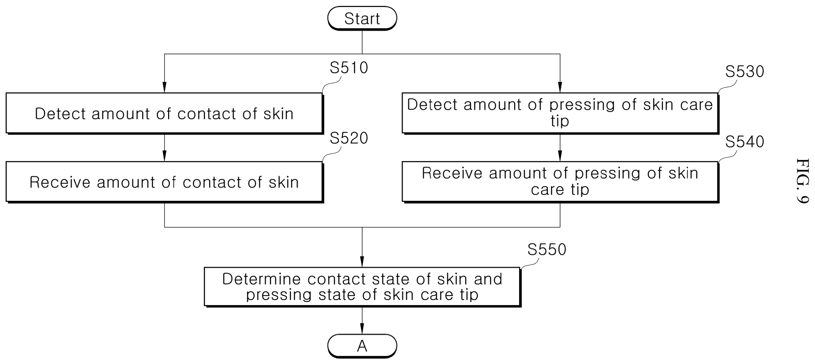

Referring to FIGS. 8 and 9, an operation method of the skin care device 1000 may include a first detection step S510, a first receiving step S520, a second detection step S530, a second receiving step S540, and a determination step S550.

As shown in FIG. 8, the first processor 132 may control the first sensing module 110 to detect the amount of contact of the skin S through the first sensing module 110 of the hand piece 100 (step S510).

The first processor 132 may receive the amount of contact of the skin S detected from the first sensing module 110 (step S520). The first processor 132 may control the second sensing module 120 to detect the amount of pressing of the skin care tip 102 through the second sensing module 120 (step S530).

The first processor 132 may receive the amount of pressing of the skin care tip 102 detected from the second sensing module 120 (step S540).

The first processor 132 may determine the contact state of the skin S and the pressing state of the skin care tip 102 based on the amount of contact and amount of pressing detected through the first sensing module 110 and the second sensing module 120 (step S550). Here, the first processor 132 may determine whether the skin S is properly contacted and whether the skin care tip 102 is properly pressed. At this time, the first processor 132 may determine whether pressure is applied to minimize the influence of the gel applied to the skin S.

As illustrated in FIG. 9, the second processor 212 may control the first sensing module 110 to detect the amount of contact of the skin S through the first sensing module 110 (step S510). The second processor 212 may receive the amount of contact of the skin S detected from the first sensing module 110 (step S520).

At the same time, the second processor 212 may control the second sensing module 120 to detect the amount of pressing of the skin care tip 102 through the second sensing module 120 (step S530). The second processor 212 may receive the amount of pressing of the skin care tip 102 detected from the second sensing module 120 (step S540).

The second processor 212 may determine the contact state of the skin S and the pressing state of the skin care tip 102 based on the detected amount of contact and the amount of pressing (step S550). Here, the second processor 212 may determine whether the skin S is properly contacted and the skin care tip 102 is properly pressed. In this case, the second processor 212 may determine whether pressure is applied to minimize the influence of the gel applied to the skin S.

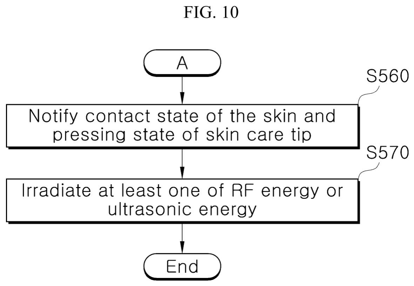

Referring to FIG. 10, the operating method of the skin care device 1000 according to the present disclosure may further include a notification step S560 and an irradiation step S570.

As an example, the first processor 132 may control the first notification module 140 to notify the determined contact state of the skin S and the determined pressing state of the skin care tip 102 (step S560).

The first processor 132 may extract at least one of a preset RF energy intensity or a preset ultrasonic energy intensity linked with the detected amount of contact and the detected amount of pressing based on the amount of contact and the amount of pressing detected through the first sensing module 110 and the second sensing module 120.

The first processor 132 may control the energy irradiation module 150 to irradiate at least one of the RF energy or the ultrasonic energy based on at least one of the extracted RF energy intensity or the ultrasonic energy intensity (step S570). In this case, the first processor 132 may extract at least one of the corresponding RF energy intensity or the corresponding ultrasonic energy intensity corresponding to the level of the amount of contact and the amount of pressing, and the energy irradiation module 150 may irradiate at least one of the RF energy or the ultrasonic energy intensity with at least one of the corresponding RF energy intensity or the corresponding ultrasonic energy intensity.

As another example, the second processor 212 may control the second notification module 220 to notify the determined contact state of the skin S and the determined pressing state of the skin care tip 102 (step S560).

The second processor 212 may extract at least one of the RF energy intensity or the ultrasonic energy intensity that are set in connection with the detected amount of contact and amount of pressing based on the amount of contact and the amount of pressing detected through the first sensing module 110 and the second sensing module 120.

The second processor 212 may control the energy irradiation module 150 to irradiate at least one of the RF energy or the ultrasonic energy based on at least one of the extracted RF energy intensity or the ultrasonic energy intensity (step S570). In this case, the second processor 212 may extract at least one of the corresponding RF energy intensity or the corresponding ultrasonic energy intensity corresponding to the level of the amount of contact and the level of the amount of pressing, and the energy irradiation module 150 may irradiate at least one of the RF energy or the ultrasonic energy with at least one of the corresponding RF energy intensity or the corresponding ultrasonic energy intensity.

Therefore, the skin care device and the method therefor according to the present disclosure may accurately determine the contact and pressing state of the skin, and prevent the occurrence of a burn accident while transmitting optimal energy to the skin.

At least one component may be added or deleted corresponding to the performance of the components illustrated in FIGS. 1 to 7. In addition, it will be easily understood by a person having ordinary knowledge in the relevant technical field that the mutual positions of the components may be changed corresponding to the performance or structure of the system.

Although FIGS. 8 to 10 describe the execution of multiple steps sequentially, this is only an exemplary description of the technical idea of the present embodiment, and a person having ordinary knowledge in the technical field to which the present embodiment belongs may modify and change the order described in FIGS. 8 to 10 without departing from the essential characteristics of the present embodiment, or may execute one or more of the multiple steps in parallel, thereby applying various modifications and variations, and thus FIGS. 8 to 10 are not limited to a chronological order.

As described above, the disclosed embodiments have been described with reference to the attached drawings. A person having ordinary knowledge in the technical field to which the present disclosure belongs will understand that the present disclosure may be implemented in a form different from the disclosed embodiments without changing the technical idea or essential features of the present disclosure. The disclosed embodiments are exemplary and should not be construed as limiting.

## Claims

1. A skin care device comprising:
a main body;
a hand piece; and
a processor configured to:
based on an amount of pressing of a skin care tip and an amount of contact with a skin detected through the hand piece,
determine a contact state of the skin and a pressing state of the skin care tip.

2. The device according to claim 1, wherein the hand piece includes:
a housing;
the skin care tip coupled to the housing;
a first sensing module provided on the skin care tip and configured to detect the amount of contact with the skin; and
a second sensing module provided on the housing or the skin care tip and configured to detect the amount of pressing of the skin care tip.

3. The device according to claim 1,
wherein the processor is provided on at least one of the hand piece or the main body, and
wherein the processor is configured to determine the contact state of the skin and the pressing state of the skin care tip based on the detected amount of contact and the amount of pressing.

4. The device according to claim 2,
wherein the first sensing module is a temperature sensor.

5. The device according to claim 2,
wherein the second sensing module is at least one of a pressure sensor or a photo interrupter sensor.

6. The device according to claim 1, further including a notification module configured to notify the determined contact state of the skin and the determined pressing state of the skin care tip.

7. The device according to claim 6,
wherein the notification module is provided in at least one of the hand piece or the main body.

8. The device according to claim 3,
wherein the processor is configured to extract at least one of a preset RF energy intensity or a preset ultrasonic energy intensity based on and linked with the detected contact amount and the detected pressing amount, and
wherein the hand piece further includes an energy irradiation module configured to irradiate at least one of the RF energy and the ultrasonic energy based on at least one of the extracted RF energy intensity and the extracted ultrasonic energy intensity.

9. A skin care method performed by a skin care device, comprising:
detecting, by a hand piece of the skin care device, an amount of pressing of a skin care tip and an amount of contact with a skin;
receiving, by the hand piece, the detected amount of pressing of the skin care tip and the detected amount of contact with the skin; and
determining, by a processor of the skin care device, the contact state of the skin and the pressing state of the skin care tip based on the detected amount of contact and the detected amount of pressing.

10. The method according to claim 9,
wherein detecting the amount of pressing of the skin care tip and the amount of contact with the skin includes:
detecting, by a first sensing module provided on the skin care tip of the hand piece, the amount of contact with the skin; and
detecting, by a second sensing module provided on the housing of the hand piece or the skin care tip, the amount of pressing of the skin care tip.

11. The method according to claim 9,
wherein determining the contact state of the skin and the pressing state of the skin care tip further includes:
determining, by the processor provided on at least one of the hand piece or a main body of the skin care device, the contact state of the skin and the pressing state of the skin care tip based on the detected amount of contact and the detected amount of pressing.

12. The method according to claim 9,
wherein detecting the amount of pressing of the skin care tip and the amount of contact with the skin includes:
detecting, by a temperature sensor of the hand piece, the amount of contact with the skin.

13. The method according to claim 9,
wherein detecting the amount of pressing of the skin care tip and the amount of contact with the skin includes:
detecting, by at least one of a pressure sensor or a photo interrupter sensor of the hand piece, the amount of pressing of the skin care tip.

14. The method according to claim 9, further comprising:
notifying, by a notification module of the skin care device, the determined contact state of the skin and the determined pressing state of the skin care tip.

15. The method according to claim 9, further comprising:
extracting, by the processor, at least one of a preset RF energy intensity or a preset ultrasonic energy intensity linked with the detected amount of contact and the detected amount of pressing; and
controlling, by the processor, an energy irradiation module of the hand piece to irradiate at least one of the RF energy or the ultrasonic energy based on at least one of the extracted RF energy intensity or the extracted ultrasonic energy intensity.
